# EUROPÄISCHE PATENTSCHRIFT

(11) **EP 2 893 957 B1**
(45) Veröffentlichungstag und Bekanntmachung des Hinweises auf die Patenterteilung: **11.07.2018**
(21) Anmeldenummer: 14199904.5
(22) Anmeldetag: 22.12.2014
(51) Int. Cl.: A61Q 11/00, A61K 8/73

(54) **Mund- und Zahnpflege- sowie Zahnreinigungsmittel**
Oral and tooth care and tooth cleaning agent
Produit de nettoyage pour dents et d'entretien de la bouche et des dents

(30) Priorität: 09.01.2014 DE 102014200255; 30.01.2014 DE 102014201628
(43) Veröffentlichungstag der Anmeldung: 15.07.2015
(73) Patentinhaber: Dr. Rudolf Liebe Nachfolger GmbH & Co. KG, 70771 Leinfelden-Echterdingen (DE)
(72) Erfinder: Quasdorff, Dr. Jens-Martin, 74343 Sachsenheim (DE)
(74) Vertreter: Gauss, Nikolai

(56) Entgegenhaltungen:
- EP-A1- 1 726 288
- WO-A2-2004/071321
- US-A- 5 158 764
- US-A- 5 496 541

## Beschreibung

Die Erfindung betrifft ein Mund- und Zahnpflege- sowie Zahnreinigungsmittel in Form einer Paste mit einem Zusatz von feinkörnigen, wasserunlöslichen Reinigungskörpern aus Zellulose.

Aus der US 5,158,764 A ist ein derartiges Mund- und Zahnpflege- sowie Zahnreinigungsmittel bekannt.

Formulierungen pastöser Mund- und Zahnpflege- sowie Zahnreinigungsmittel enthalten oftmals einen Zusatz von Reinigungskörpern in Form von Silikat- oder Kieselgelteilchen. Die als Schleifmittel wirkenden Reinigungskörper sollen vor allem zur Bekämpfung der Plaque-Bildung auf den Zähnen und damit zur Karies-Prophylaxe dienen. Aufgrund ihrer Härte und ihrer kantigen Oberflächenstruktur besitzen die Schleifmittel jedoch einen hohen Abriebwert und greifen deshalb den Zahnschmelz, vor allem bei sehr häufiger Zahnpflege, verhältnismäßig stark an. Weiterhin können die beim Zähneputzen mit der Zahnbürste nur schwer zugänglichen Stellen, wie beispielsweise die Oberflächen von Multibandapparaturen versehenen Zähnen oder die Kontaktstellen zwischen den Zähnen, mit den bekannten Mund- und Zahnpflege- sowie Zahnreinigungsmitteln nur unzureichend gereinigt werden. Die nicht entfernten Speisereste können langfristig kariöse Defekte an den Zähnen verursachen.

In der DE 43 42 356 B4 wird vorgeschlagen, in einer Zahncreme für die Zahnreinigung und die Mund- und Zahnpflege als Bestandteil Kügelchen aus wasserunlöslichem synthetischem Ethylen-Vinylacetat-Copolymerisat vorzusehen. Die Kügelchen bei diesem Mund- und Zahnpflege- sowie Zahnreinigungsmittel wirken hier als Reinigungskörper, die beim Zähneputzen auch an die nicht direkt von den Borsten einer Zahnbürste erreichbaren Bereiche gelangen. Aufgrund des großen Härteunterschieds zwischen diesen Kügelchen und einem Zahn wird damit eine Schädigung des Zahnschmelzes bzw. des Zahnbeins bei freiliegenden Zahnhälsen beim Zähneputzen vermieden.

Hiervon ausgehend liegt der Erfindung die Aufgabe zugrunde, ein Mittel für die Zahnreinigung und die Mund- und Zahnpflege bereitzustellen, das auch in schwer zugänglichen Zahnbereichen eine gute und gleichzeitig schonende Zahnreinigung ermöglicht und das als Bestandteile weitgehend natürliche Stoffe enthält.

Zur Lösung dieser Aufgabe werden gemäß der Erfindung die in Anspruch 1 angegebenen Merkmalskombinationen vorgeschlagen. Weitere vorteilhafte Ausgestaltungen und Weiterbildungen der Erfindung ergeben sich aus den abhängigen Ansprüchen.

Die Erfindung beruht auf der Erkenntnis, dass auch der Zusatz von feinkörnigen, wasserunlöslichen Reinigungskörpern aus Zellstoff in einer Paste für die Zahnreinigung und die Mund- und Zahnpflege die Zähne von schädlichem Zahnbelag insbesondere in engen Zahnzwischenräumen und in Bereichen unter dem Zahnfleischsaum beim Zähneputzen befreien kann, ohne dass dabei der Zahnschmelz angegriffen wird, was z. B. Messungen des RDA-Werts (Radioactive Dentine Abrasion) zeigen.

Der Erfindung liegt insbesondere der Gedanke zugrunde, dass sich die Härte des aus natürlichen Pflanzenfasern gewonnenen Zellstoffs über einen weiten Bereich variieren und so auf eine für Reinigungskörper in einem Mund- und Zahnpflege- sowie Zahnreinigungsmittel günstige Härte einstellen lässt, bei der der Zahnschmelz während der Anwendung eines solchen Mittels geschont wird. Ein Vorteil der aus Zellstoff bestehenden Reinigungskörper ist auch, dass diese keinerlei Toxizität oder Reizwirkung besitzen. Solche Reinigungskörper sind in Abwässern außerdem gut biologisch abbaubar.

Bevorzugt ist der Zellstoff der Reinigungskörper in dem erfindungsgemäßen Mittel für die Zahnreinigung und die Mund- und Zahnpflege mikrokristalline Zellulose, die z. B. in der pharmazeutischen Industrie als Trennmittel oder als Trägerstoff für Wirkstoffe eingesetzt wird.

Erfindungsgemäß haben die Reinigungskörper eine im Wesentlichen kugelige, runde Geometrie nach der Art einer Kartoffel. Die Struktur dieser Reinigungskörper ist also nicht scharfkantig, so dass bei der Reinigung von Zähnen mit dem erfindungsgemäßen Mund- und Zahnpflege- sowie Zahnreinigungsmittel keine oder nur geringe Abrasion von Zahnschmelz auftritt. Die Oberfläche hat hierzu z. B. lokale Krümmungsradien r, für die im Mittel auf einem jeden Oberflächenelement der Ausdehnung 10 µm × 10 µm gilt: r ≥ 1µm, bevorzugt r ≥ 10 µm; besonders bevorzugt r ≥ 50µm.

Für eine gute Reinigungswirkung ist ein Anteil der Reinigungskörper in der Paste für die Zahnreinigung und die Mund- und Zahnpflege von 5 bis 25 Gew.-% von Vorteil. Aufgrund der damit bewirkten Dichte der Reinigungskörper in der Paste wird erreicht, dass die von den Borsten einer Zahnbürste angetriebenen Reinigungskörper nach Art eines Reibradgetriebes Schub- und Rotationskörper an benachbarte Reinigungskörper vermitteln. Hierdurch können die Reinigungskörper auch an den nicht direkt von den Borsten der Zahnbürste erreichbaren Bereiche an der Oberfläche eines Zahns in Bewegung versetzt werden.

Die Reinigungskörper aus Zellstoff haben erfindungsgemäß eine bevorzugt glatte Oberfläche mit einer unregelmäßigen Knollenstruktur. Damit wird erreicht, dass die Oberfläche der Reinigungskörper an die Geometrie einer Vielzahl von Erhebungen und Vertiefungen sowie an die Fissuren von Zähnen angepasst ist. Das gewährleistet, dass beim Zähneputzen auch dort eine gute Reinigungswirkung erzielt wird.

Für die Reinigungskörper ist der Durchmesser 200 µm ≤ D ≤ 600 µm von Vorteil, vorzugsweise gilt für den Durchmesser 300 µm ≤ D ≤ 500 µm, weiter vorzugsweise 350 µm ≤ D ≤ 450 µm. Insbesondere folgende Durchmesser der Reinigungskörper haben sich im Hinblick auf die Eigenschaft der guten und gleichzeitig schonenden Zahnreinigung des Mund- und Zahnpflege- sowie Zahnreinigungsmittels der Erfindung als günstig erwiesen: D ≈ 300 µm, D ≈ 350 µm, D ≈ 400 µm und D ≈ 450 µm. Unter dem Durchmesser D wird dabei vorliegend der größtmögliche Abstand zweier Punkte auf der Oberfläche des Reinigungskörpers verstanden. Solche Reinigungskörper eignen sich insbesondere zur Reinigung von mit Multibandapparaturen versehenen Zähnen.

Das erfindungsgemäße Mund- und Zahnpflege- sowie Zahnreinigungsmittel enthält bevorzugt einen Wirkstoff oder auch mehrere Wirkstoffe, z. B. Bisabolol und/oder Panthenol. Ein erfindungsgemäßes Mund- und Zahnpflege- sowie Zahnreinigungsmittel kann als Bestandteil auch Aminofluorid und/oder Natriumfluorid und/oder ethanolisch wässrigen Extrakt aus Kamilleblütenköpfen und/oder Kokosfettsäure-Amido-Propyl-Betain und/oder Glycerin und/oder Wasser und/oder Propylenglycol und/oder Natriummethaphosphat und/oder Kieselsäure und/oder Titandioxid und/oder Saccharin und/oder Aromastoffe enthalten. Die Wirkstoffe in dem Mund- und Zahnpflege- sowie Zahnreinigungsmittel sind bevorzugt in einem Lösemittel gelöst, insbesondere in einem ätherischen Öl, wie z. B. Menthol, Eucalyptol, Anethol, Eugenol, d-Limonen und/oder Citronellol.

Eine Erkenntnis der Erfindung ist es auch, dass Reinigungskörper aus Zellstoff in einem erfindungsgemäßen Mund- und Zahnpflege- sowie Zahnreinigungsmittel sehr viel weniger Wirkstofflösung inkorporieren als dies bei Reinigungskörpern aus synthetischem Ethylen-Vinylacetat-Copolymerisat in einem herkömmlichen Mund- und Zahnpflege- sowie Zahnreinigungsmittel der Fall ist.

In Versuchen hat der Erfinder nämlich herausgefunden, dass Reinigungskörper aus Zellstoff bei Wasser- und Ölzusatz nicht aufquellen. Insbesondere hat der Erfinder herausgefunden, dass 150g Wirkstofflösung von 100g Reinigungskörpern aus synthetischem Ethylen-Vinylacetat-Copolymerisat mit dem Durchmesser 200 µm ≤ D ≤ 600 µm innerhalb von 3 Tagen vollständig aufgesogen wird, wogegen Reinigungskörper aus mikrokristalliner Zellulose eine solche Wirkstofflösung nicht aufnehmen.

Anders als Reinigungskörpern aus synthetischem Ethylen-Vinylacetat-Copolymerisat entziehen also die Reinigungskörper aus Zellstoff in einem erfindungsgemäßen Mund- und Zahnpflege- sowie Zahnreinigungsmittel der Paste keine Wirkstoffe. Vielmehr gewährleisten die Reinigungskörper aus Zellstoff, dass die in dem Mund- und Zahnpflege- sowie Zahnreinigungsmittel enthaltenen Wirkstoffe beim Zähneputzen zu den Zähnen, in das Zahnfleisch und in die Mundschleimhaut gelangen können. Eine Erkenntnis der Erfindung ist es auch, dass die Reinigungskörper aus Zellstoff zu einer guten Homogenität des erfindungsgemäßen Mund-, Zahnpflege- sowie Zahnreinigungsmittels beitragen.

Indem das Mund- und Zahnpflege- sowie Zahnreinigungsmittel den Wirkstoff Xylitol enthält, können kariogene Bakterien gehemmt und es kann damit auch die Bildung von Zahnbelag verzögert werden.

Eine vorteilhafte Formulierung eines erfindungsgemäßen Mund- und Zahnpflege- sowie Zahnreinigungsmittels mit einer großen Homogenität ist wie folgt:

| | |
|---|---|
| 50 Gew.-% | Feuchthaltemittel |
| 20 Gew.-% | Reinigungskörper aus mikrokristalliner Zellulose mit einem Durchmesser von bevorzugt 400 µm |
| 25 Gew.-% | Schleifmittel (Kieselsäure) |
| 2 Gew.-% | Binde- und Verdickungsmittel (Carboxyethyl-Zellulose) |
| 1,5 Gew.-% | Schaumbildner (Tenside) |
| 0,9 Gew.-% | Wirkstoffe |
| 0,3 Gew.-% | Aromatisierungsmittel |
| 0,1 Gew.-% | Fluoride |

Das vorstehend beschriebene Mund- und Zahnpflege- sowie Zahnreinigungsmittel eignet sich insbesondere für die Reinigung von mit Multibandapparaturen versehenen Zähnen.

Die Reinigungskörper aus mikrokristalliner Zellulose in dem erfindungsgemäßen Mund- und Zahnpflege sowie Zahnreinigungsmittel können insbesondere die geometrische Form von mikrokristalliner Zellulose Typ VIVAPUR® CS 300 S, Typ VIVAPUR® CS 350 S, Typ VIVAPUR® CS 400, Typ VIVAPUR® CS 450 S oder auch Typ VIVAPUR® CS 500 der Firma Rettenmaier & Söhne aus D-73494 Rosenberg haben.

Im Folgenden wird die Erfindung anhand der Zeichnung näher erläutert. Es zeigen:
- Fig. 1: einen mittels eines Mund- und Zahnpflege- sowie Zahnreinigungsmittels auf einen mit einer Multibandapparatur versehenen Zahn einwirkende Zahnbürste;
- Fig. 2: eine ausschnittsweise vergrößerte Darstellung der Fig. 1;
- Fig. 3: einen einzelnen Reinigungskörper in dem Mund- und Zahnpflegesowie Zahnreinigungsmittel; und
- Fig. 4: eine vergrößerte Ansicht eines Oberflächenabschnitts des Reinigungskörpers der Fig. 3.

Die folgende Beschreibung bezieht sich auf die Anwendung einer Zahnpaste (Zahncreme), die folgende Formulierung aufweist:

| | |
|---|---|
| 50 Gew.-% | Feuchthaltemittel |
| 20 Gew.-% | Reinigungskörper aus mikrokristalliner Zellulose mit einem Durchmesser von bevorzugt 400 µm |
| 25 Gew.-% | Schleifmittel (Kieselsäure) |
| 2 Gew.-% | Binde- und Verdickungsmittel (Carboxyethyl-Zellulose) |
| 1,5 Gew.-% | Schaumbildner (Tenside) |
| 0,9 Gew.-% | Wirkstoffe |
| 0,3 Gew.-% | Aromatisierungsmittel |
| 0,1 Gew.-% | Fluoride |

Diese Zahnpaste ist ein Mund- und Zahnpflege- sowie Zahnreinigungsmittel. Die darin enthaltenen Reinigungskörper besitzen einen mittleren Teilchendurchmesser von etwa 400 µm. Sie haben eine im Wesentlichen kugelige Gestalt mit einer glatten Oberfläche, die eine unregelmäßige Knollenstruktur hat.

Die Zahnpaste eignet sich zur Reinigung von mit Multibandapparaturen versehenen Zähnen. Derartige Apparaturen, die zur Korrektur von Zahnfehlstellungen dienen, weisen auf die Zähne aufgeklebte mechanische Aufnehmer (Brackets) auf, an denen Drahtbögen (Loops) angebracht und arretiert werden können.

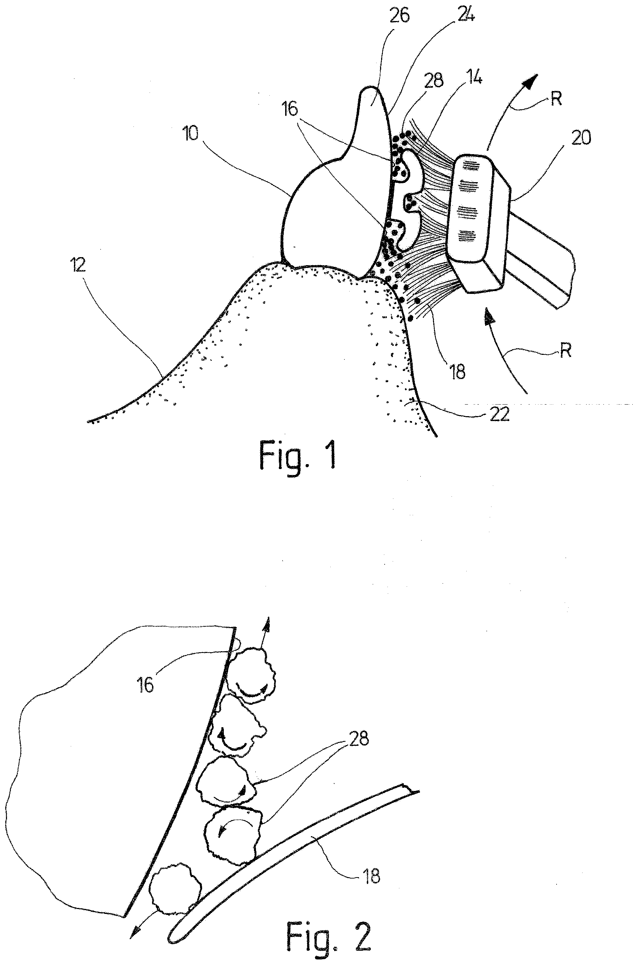

Zur Veranschaulichung der Reinigungswirkung der Zahnpaste ist in Fig. 1 ein Vorderzahn 10 eines Unterkiefers 12 mit einem aufgeklebten Aufnehmer 14 einer Multibandapparatur während des Reinigungsvorgangs dargestellt. An den von dem Aufnehmer 14 übergriffenen Zahnbereichen 16 können sich aus Speiseresten bestehende Ablagerungen bilden, die bei gewöhnlicher Zahnreinigung mit den Borsten 18 einer Zahnbürste 20 kaum erreichbar sind.

Die mit den eine im Wesentlichen kugelige Gestalt aufweisenden Reinigungskörpern 28 versehene Zahnpaste wird mittels einer üblichen Putztechnik, beispielsweise mit der Rotationsmethode angewendet. Dazu wird die Zahnpaste mit den Borsten 18 der Zahnbürste 20 in einer kreisenden Bewegung (Pfeil R) vom Zahnfleisch 22 her über die Zahnoberfläche 24 bzw. über die Multibandapparatur und deren Aufnehmer 14 in Richtung Zahnspitze 26 geführt. Durch die Einwirkung der Speichelflüssigkeit und das thixotrope Verhalten der Zahnpaste werden die in der Zahnpaste enthaltenen wasserunlöslichen Reinigungskörper 28 beweglich.

Die von den Borsten 18 der Zahnbürste 20 angetriebenen Reinigungskörper 28 vermitteln nach Art eines Reibradgetriebes Schub- und Rotationsbewegungen an benachbarte Reinigungskörper 28, wie in Fig. 2 veranschaulicht. Dabei gelangen die Reinigungskörper 28 auch an die nicht direkt von den Borsten 18 erreichbaren Bereiche 16.

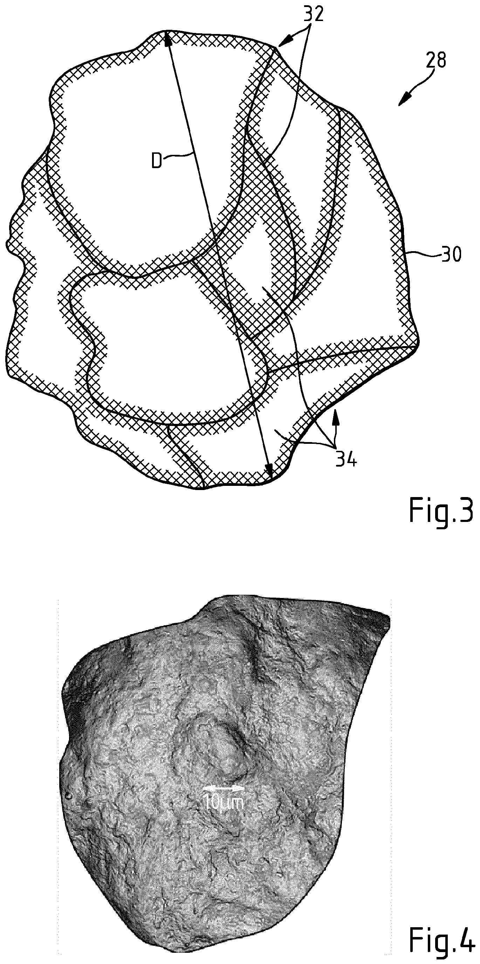

Die Fig. 3 zeigt einen in der Zahnpaste enthaltenen Reinigungskörper 28 aus mikrokrostalliner Zellulose in der Form von mikrokrostalliner Zellulose Typ VIVAPUR® CS 400 S der Firma Rettenmaier & Söhne in Vergrößerung. Der Durchmesser D der Reinigungskörper 28 in der Zahnpaste beträgt etwa 400 µm. Dabei ist unter dem Durchmesser D der größtmögliche Abstand zweier Punkte auf der Oberfläche des Reinigungskörpers zu verstehen. In der Fig. 4 ist ein Abschnitt der Oberfläche des Reinigungskörpers 28 mit etwa 400-facher Vergrößerung gezeigt. Die Oberfläche 30 der Reinigungskörper 28 ist glatt. Die Oberfläche 30 hat jedoch eine Knollenstruktur ähnlich einer Kartoffel mit zahlreichen lokalen Erhebungen 32 und Vertiefungen 34. Die Oberfläche hat lokale Krümmungsradien r, für die im Mittel auf einem jeden Oberflächenelement der Ausdehnung 10 µm × 10 µm gilt: r ≥ 1µm.

Die Reinigungswirkung der Reinigungskörper 28 in der Zahnpaste besteht zum einen darin, dass die Rotationsbewegung zu einer Politur der Zahnoberfläche 24 führt. Zum anderen dringen die Reinigungskörper direkt in die gröberen Verschmutzungen bzw. Ablagerungen ein und vergrößern deren Oberfläche. Dies führt zu einem Aufbrechen der Ablagerungen und zu einer erhöhten Wasserlöslichkeit, wodurch sich die Ablagerungen leichter entfernen lassen.

Die insbesondere in der Fig. 4 sichtbare Knollenstruktur der Oberfläche der Reinigungskörper 28 mit der darin ausgebildeten Vielzahl von Erhebungen und Vertiefungen bewirkt, dass beim Zähneputzen mit der Zahnpaste die Reinigungskörper auch in engen Fissuren mit der Oberfläche der Zähne in Kontakt geraten, so dass beim Zähneputzen dort ebenfalls eine gute Reinigungswirkung erzielt wird.

Durch die Anwendung der Reinigungskörper 28, die sich beim Zähneputzen gleichsam als Kugellager zwischen dem Führungsbogen aus Draht und den an den Zähnen befestigten Aufnehmern (Brackets) einer Multibandapparatur ansiedeln, wird eine erhebliche Reibungsverminderung erreicht, so dass eine zusätzliche Kraft für das Bewegen der Reinigungskörper 28 an den Zähnen zur Verfügung steht. Das Abrollen der Reinigungskörper 28 hält dabei den Abrieb des Dentins beim Zähneputzen gering. Trotz der geringen Abrasivität des Mund-, Zahnpflege- und Zahnreinigungsmittels werden die Zähne damit bestens gereinigt und sind dann zungenglatt.

Im Rahmen von Untersuchungen hat der Erfinder festgestellt, dass die plaque-reduzierende Reinigungswirkung der vorstehend beschriebenen Zahnpasta der in der DE 43 42 356 B4 angegebenen Reinigungswirkung der dort beschriebenen Zahnpasta grundsätzlich entspricht.

Zusammenfassend ist insbesondere folgendes festzuhalten: Die Erfindung betrifft ein Mund- und Zahnpflege- sowie Zahnreinigungsmittel in Form einer Paste mit einem Zusatz von feinkörnigen, wasserunlöslichen Reinigungskörpern 28. Die Reinigungskörper bestehen aus einem Zellstoff.

### Bezugszeichenliste

- 10: Vorderzahn
- 12: Unterkiefer
- 14: Aufnehmer
- 16: Zahnbereich
- 18: Borsten
- 20: Zahnbürste
- 22: Zahnfleisch
- 24: Zahnoberfläche
- 26: Zahnspitze
- 28: Reinigungskörper
- 30: Oberfläche
- 32: Erhebung
- 34: Vertiefung

## Patentansprüche

1. Mund- und Zahnpflege- sowie Zahnreinigungsmittel in Form einer Paste mit einem Zusatz von feinkörnigen, wasserunlöslichen Reinigungskörpern (28) aus Zellulose, wobei der Anteil der Reinigungskörper in der Paste 5 bis 25 Gew.-% beträgt,
**dadurch gekennzeichnet, dass**
die Zellulose mikrokristalline Zellulose ist und die Reinigungskörper eine kugelige, runde Geometrie mit einer Oberfläche mit einer unregelmäßigen Knollenstruktur haben und dabei lokale Krümmungsradien aufweisen, für die im Mittel auf einem jeden Oberflächenelement der Ausdehnung 10 µm × 10 µm gilt: r ≥ 1 µm, wobei für den Durchmesser D der Reinigungskörper gilt: 300 µm ≤ D ≤ 500 µm, wobei unter dem Durchmesser der Reinigungskörper der größtmögliche Abstand zweier Punkte auf der Oberfläche zu verstehen ist, und wobei die Paste ein ätherisches Öl aus der Gruppe Menthol, Eucalyptol, Anethol, Eugenol, d-Limonen Citronellol mit einem darin aufgenommenen Wirkstoff enthält.

2. Mittel nach Anspruch 1, **dadurch gekennzeichnet, dass** für die lokalen Krümmungsradien im Mittel auf einem jeden Oberflächenelement der Ausdehnung 10 µm × 10 µm gilt: r ≥ 10 µm.

3. Mittel nach Anspruch 1, **dadurch gekennzeichnet, dass** für die lokalen Krümmungsradien im Mittel auf einem jeden Oberflächenelement der Ausdehnung 10 µm × 10 µm gilt: r ≥ 50 µm.

4. Mittel nach einem der Ansprüche 1 bis 3, **dadurch gekennzeichnet, dass** für den Durchmesser D der Reinigungskörper gilt: D ≈ 400 µm.

5. Mittel nach einem der Ansprüche 1 bis 4, **gekennzeichnet durch** mehrere Wirkstoffe.

6. Mittel nach Anspruch 5, **dadurch gekennzeichnet, dass** die mehreren Wirkstoffe in einem in der Paste enthaltenen ätherischen Öl aufgenommen sind.

7. Mittel nach Anspruch 5 oder 6, **gekennzeichnet durch** den Wirkstoff Xylitol.

8. Mittel nach einem der Ansprüche 1 bis 7, **gekennzeichnet durch** folgende Bestandteile
| | |
|---|---|
| 50 Gew.-% | Feuchthaltemittel |
| 20 Gew.-% | Reinigungskörper aus mikrokristalliner Zellulose |
| 25 Gew.-% | Schleifmittel |
| 2 Gew.-% | Binde- und Verdickungsmittel |
| 1,5 Gew.-% | Schaumbildner |
| 0,9 Gew.-% | Wirkstoffe |
| 0,3 Gew.-% | Aromatisierungsmittel |
| 0,1 Gew.-% | Fluoride |

9. Mittel nach Anspruch 8, **dadurch gekennzeichnet, dass** das Schleifmittel Kieselsäure ist.

10. Mittel nach Anspruch 8 oder 9, **dadurch gekennzeichnet, dass** das Binde- und Verdickungsmittel Carboxyethyl-Zellulose ist.

11. Mittel nach einem der Ansprüche 9 oder 10, **dadurch gekennzeichnet, dass** die Schaumbildner Tenside sind.

## Claims

1. Mouth and tooth care and tooth cleaning composition in the form of a paste comprising an addition of fine-grained, water insoluble cleaning bodies (28) composed of cellulose, wherein the proportion of the cleaning bodies in the paste is 5% to 25% by weight,
**characterized in that**
the cellulose is microcrystalline cellulose and the cleaning bodies have a spherical, round geometry with a surface having an irregular nodular structure and thus have local radii of curvature for which, on average on every surface element having dimensions of 10 µm x 10 µm, r ≥ 1 µm, wherein for the diameter D of the cleaning bodies 300 µm ≤ D ≤ 500 µm, wherein the diameter of the cleaning bodies is to be understood as meaning the greatest possible distance between two points on the surface and wherein the paste contains an essential oil from the group of menthol, eucalyptol, anethol, eugenol, d-limonene, citronellol with an active ingredient absorbed therein.

2. Composition according to Claim 1, **characterized in that** for the local radii of curvature, on average on every surface element having dimensions of 10 µm x 10 µm, r ≥ 10 µm.

3. Composition according to any of Claims 1 to 3, **characterized in that** for the local radii of curvature, on average on every surface element having dimensions of 10 µm x 10 µm, r ≥ 50 µm.

4. Composition according to any of Claims 1 to 3, **characterized in that** for the diameter D of the cleaning bodies D≈400 µm.

5. Composition according to any of Claims 1 to 4, **characterized by** multiple active ingredients.

6. Composition according to Claim 5, **characterized in that** the multiple active ingredients are absorbed in an essential oil present in the paste.

7. Composition according to Claim 5 or 6, **characterized by** the active ingredient xylitol.

8. Composition according to any of Claims 1 to 7, **characterized by** the following constituents
50% by weight of humectant
20% by weight of cleaning bodies composed of microcrystalline cellulose
25% by weight of abrasive
2% by weight of binding and thickening agent
1.5% by weight of foaming agents
0.9% by weight of active ingredients
0.3% by weight of aromas
0.1% by weight of fluorides

9. Composition according to Claim 8, **characterized in that** the abrasive is silica.

10. Composition according to Claim 8 or 9, **characterized in that** the binding and thickening agent is carboxyethylcellulose.

11. Composition according to either of Claims 9 and 10, **characterized in that** the foaming agents are surfactants.

## Revendications

1. Agent de nettoyage pour les dents et d'entretien de la bouche et des dents sous la forme d'une pâte contenant un ajout de corps nettoyants (28) de granulométrie fine, insolubles dans l'eau, en cellulose, la proportion des corps nettoyants dans la pâte étant de 5 à 25 % en poids,
**caractérisé en ce que**
la cellulose est de la cellulose microcristalline et les corps nettoyants ont une géométrie ronde sphérique avec une surface à structure tuberculeuse irrégulière et présentent ainsi des rayons de courbure locaux pour lesquels, en moyenne pour chaque élément de surface d'une dimension 10 µm x 10 µm : r ≥ 1 µm, avec pour le diamètre D des corps nettoyants : 300 µm ≤ D ≤ 500 µm, le diamètre des corps nettoyants se rapportant à l'écart le plus grand possible entre deux points sur la surface, et la pâte contenant une huile éthérée du groupe constitué par le menthol, l'eucalyptol, l'anéthol, l'eugénol, le d-limonène, le citronellol avec un agent actif absorbé dans celle-ci.

2. Agent selon la revendication 1, **caractérisé en ce que** pour les rayons de courbure locaux, en moyenne pour chaque élément de surface de dimension 10 µm x 10 µm : r ≥ 10 µm.

3. Agent selon la revendication 1, **caractérisé en ce que** pour les rayons de courbure locaux, en moyenne pour chaque élément de surface de dimension 10 µm x 10 µm : r ≥ 50 µm.

4. Agent selon l'une quelconque des revendications 1 à 3, **caractérisé en ce que** pour le diamètre D des corps nettoyants : D ≈ 400 µm.

5. Agent selon l'une quelconque des revendications 1 à 4, **caractérisé par** plusieurs agents actifs.

6. Agent selon la revendication 5, **caractérisé en ce que** lesdits agents actifs sont absorbés dans une huile éthérée contenue dans la pâte.

7. Agent selon la revendication 5 ou 6, **caractérisé par** l'agent actif xylitol.

8. Agent selon l'une quelconque des revendications 1 à 7, **caractérisé par** les constituants suivants :
50 % en poids d'agents de rétention de l'humidité,
20 % en poids de corps nettoyants en cellulose microcristalline,
25 % en poids d'agents abrasifs,
2 % en poids de liants et d'épaississants,
1,5 % en poids d'agents moussants,
0,9 % en poids d'agents actifs,
0,3 % en poids d'aromatisants,
0,1 % en poids de fluorures.

9. Agent selon la revendication 8, **caractérisé en ce que** l'agent abrasif est de la silice.

10. Agent selon la revendication 8 ou 9, **caractérisé en ce que** le liant et épaississant est la carboxyéthyl-cellulose.

11. Agent selon l'une quelconque des revendications 9 ou 10, **caractérisé en ce que** les agents moussants sont des tensioactifs.
